# EUROPÄISCHE PATENTSCHRIFT

(11) **EP 2 830 550 B1**
(45) Veröffentlichungstag und Bekanntmachung des Hinweises auf die Patenterteilung: **26.08.2015**
(21) Anmeldenummer: 12718579.1
(22) Anmeldetag: 26.03.2012
(51) Int. Cl.: A61F 5/56

(54) **OKKLUSIONSSCHIENENANORDNUNG**
OCCLUSION SPLINT ARRANGEMENT
SYSTÈME DE GOUTTIÈRE OCCLUSALE

(43) Veröffentlichungstag der Anmeldung: 04.02.2015
(73) Patentinhaber: Hofmann, Konrad, 97291 Thüngersheim (DE)
(72) Erfinder: Hofmann, Konrad, 97291 Thüngersheim (DE)
(74) Vertreter: advotec.
(86) Internationale Anmeldenummer: PCT/DE2012/000314
(87) Internationale Veröffentlichungsnummer: WO 2013/143511

(56) Entgegenhaltungen:
- DE-T5-112009 001 742
- US-A1- 2005 028 826
- US-A1- 2011 155 144
- US-B1- 6 516 805

## Beschreibung

Die Erfindung betrifft eine Okklusionsschienenanordnung, insbesondere zur Schlafapnoe-Therapie, mit einer maxillaren und einer mandibularen Miniplastschiene gemäß dem Oberbegriff des Patentanspruchs 1.

Insbesondere zur Schlafapnoe-Therapie sind aus dem Stand der Technik verschiedenste Ausführungsformen von Okklusionsschienenanordnungen bekannt. Diese zielen grundlegend darauf ab, die Stellung des Oberkiefers relativ zum Unterkiefer dahingehend zu beeinflussen, dass im tiefen Schlaf ein Zurücksacken des Unterkiefers sowie des Zungenmuskels und Gaumensegels in zulässigem Maße begrenzt wird, so dass die Atemwege offengehalten werden.

Eine hierfür besonders geeignete Ausführungsform offenbart die Schrift DE 103 41 260 A1. Diese weist eine maxillare Miniplastschiene sowie eine mandibulare Miniplastschiene auf, welche jeweils auf den entsprechenden Zahnreihen aufgesteckt werden können. Weiterhin weisen diese Miniplastschienen gegenüberliegend Anlageflächen auf, so dass diese gegeneinander zur Anlage gebracht werden können. Zur Realisierung der geforderten relativen Stellung der Miniplastschienen zueinander weist eine Miniplastschiene eine Verstelleinrichtung mit einem Raststift sowie die andere Miniplastschiene eine komplementäre Rastführung auf. Durch Eingriff des Raststifts in der Rastführung wird die Position der mandibularen Miniplastschiene relativ zur maxillaren Miniplastschiene festgelegt.

Weiterhin weist die zuvor benannte Druckschrift, wie ebenso alternative Ausführungsformen, eine Verstellvorrichtung auf. Mittels dieser kann die individuelle Stellung der mandibularen Miniplastschiene relativ zur maxillaren Miniplastschiene beeinflusst werden. Dies erfolgt im vorbenannten Fall dergestalt, dass mittels einer Einstellschraube die Stellung des Raststifts in der Verstelleinrichtung verstellt werden kann.

Wenngleich mit bekannten Ausführungsformen aus dem Stand der Technik eine zuverlässige Positionierung zwischen der maxillaren und der mandibularen Miniplastschiene zur Therapie des Schlafapnoe-Syndroms ermöglicht ist, weisen diese jedoch jeweils für sich verschiedenste Nachteile auf. Dies betrifft zum einen den unnötig komplexen Aufbau und die hiermit verbundenen hohen Herstellkosten. Weiterhin besonders nachteilig ist die Anfälligkeit für Schmutzablagerungen an schwer zu reinigenden Stellen, so dass diese aus hygienischer Sicht problematisch sind bzw. einer entsprechenden chemischen Reinigung nach jeder Verwendung bedürfen.

Die US 2011/0155144 A1 beschreibt eine Okklusionsschienenanordnung bei der die mandibulare und die maxillare Miniplastschiene durch zwei Gelenkhebel gelenkig miteinander verbunden sind und nicht voneinander getrennt werden können. Die Anpassung an den Ober- und Unterkiefer erfolgt mittels einer thermoplastischen Füllmasse.

Die US 6 516 805 B1 beschreibt eine Okklusionsschienenanordnung, bei der die mandibulare und die maxillare Miniplastschiene mittels eines nach unten überstehenden Fixiervorsprungs relativ zueinander fixiert werden können. Der Fixiervorsprung ist dabei in einer Nutführung auswechselbar gelagert und wird mit einer Fixierschraube arretiert.

Die US 2005/0028826 A1 beschreibt eine Okklusionsschienenanordnung, bei der die mandibulare und die maxillare Miniplastschiene mit lösbar befestigten Positioniermitteln, zur Positionierung der Miniplastschienen relativ zueinander versehen sind. Zur Befestigung der Positioniermittel dienen dabei in den Kunststoff der Miniplastschienen eingebettete Befestigungsplatten, an denen jeweils zwei kreisrunde Befestigungsdrähte befestigt sind. Nach dem Einbetten der Befestigungsplatten in den Kunststoff der Miniplastschienen stehen die Befestigungsdrähte seitlich von der Miniplastschiene ab, und die Positioniermittel werden seitlich auf die Befestigungsdrähte aufgeschoben. Problematisch dabei ist es, dass die Befestigung der Positioniermittel an den Miniplastschienen nicht ausreichend sicher ist. Wird aber ein Positioniermittel durch unbewusste Kieferbewegungen, beispielsweise im Schlaf, von den Befestigungsdrähten heruntergeschoben, so besteht die Gefahr, dass das lose Positioniermittel verschluckt oder eingeatmet wird, was eine erhebliche Gesundheitsgefährdung darstellt.

Aufgabe der vorliegenden Erfindung ist es daher, eine Okklusionsschienenanordnung bereitzustellen, mittels der eine absolut sichere Zuordnung zwischen der mandibularen und der maxillaren Miniplastschiene ermöglicht wird und hierbei die Komplexität gegenüber bekannten Ausführungsformen reduziert werden kann.

Die gestellte Aufgabe wird durch eine erfindungsgemäße Ausführungsform nach dem Anspruch 1 gelöst.

Vorteilhafte Ausführungsformen sind Gegenstand der Unteransprüche.

Die Okklusionsschienenanordnung dient insbesondere zur Schlafapnoe-Therapie. Wenngleich somit die vorliegende Ausführungsform einer Okklusionsschienenanordnung primär zur Therapierung des Schlafapnoe-Syndroms vorgesehen ist, so ist es jedoch unbenommen, die erfindungsgemäße Ausführungsform ebenso für eine anderweitige Okklusionsschienenanordnung einzusetzen. Hierbei kann die Okklusionsschienenanordnung ebenso vorgesehen sein zur Behandlung von Gebissfehlstellungen oder als Aufbissschiene zur Verhinderung eines nächtlichen Knirschens oder dergleichen.

Die gattungsgemäße Okklusionsschienenanordnung weist eine auf der maxillaren Zahnreihe anordenbare maxillare Miniplastschiene sowie eine auf der mandibularen Zahnreihe anordenbare mandibulare Miniplastschiene auf. Verwendungsgemäß kann hierbei die maxillare Miniplastschiene gegen die mandibulare Miniplastschiene zur Anlage gebracht werden. Zur Positionierung der Miniplastschienen zueinander weist die maxillare Miniplastschiene zumindest ein maxillares Positionierungsmittel sowie die mandibulare Miniplastschiene zumindest ein mandibulares Positionierungsmittel auf. Hierbei ist die Art, Ausführung und Anordnung der Positionierungsmittel an den Miniplastschienen zunächst unerheblich. Zumindest erfordert die Positionierung der Miniplastschienen relativ zueinander einen Formschluss zwischen dem maxillaren und mandibularen Positionierungsmittel, wodurch die Relativstellung der Miniplastschienen zueinander in einer Längsrichtung und/oder in einer Querrichtung definierbar ist.

Diesbezüglich ist es gleichfalls zunächst unerheblich, ob der Formschluss beispielsweise vor einem Einsetzen auf den Zahnreihen bereits außerhalb des Munds durch Fügen der Miniplastschienen aneinander hergestellt wird, oder ob ein Formschluss erst in dem Fall auftritt, wenn die auf den Zahnreihen aufgesetzten Miniplastschienen durch ein Schließen des Munds in Kontakt zueinander gebracht werden. Zumindest wird spätestens beim Schließen des Gebisses durch Anlage der Miniplastschienen aneinander ein entsprechender Formschluss zwischen den Positionierungsmitteln bewirkt, so dass die relative Stellung der Miniplastschienen zueinander definiert wird. Diese relative Stellung kann hierbei sowohl die Längsrichtung, d.h. eine Erstreckung nach vorne bzw. nach hinten aus Sicht des Patienten, oder alternativ oder zugleich eine relative Positionierung in einer Querrichtung umfassen. Diesbezüglich ist es für die erfindungsgemäße Ausführungsform weiterhin zunächst unerheblich, ob hierbei ein geringfügiges Spiel vorgesehen ist.

Hinsichtlich der benannten Richtungen ist auszuführen, dass aus Sicht des Patienten sich die Längsrichtung entlang einer Linie von hinten nach vorne erstreckt, wobei die Hochrichtung näherungsweise senkrecht zur Okklusionsebene liegt und des Weiteren die Querrichtung entsprechend senkrecht auf der Hochrichtung sowie senkrecht zur Längsrichtung liegt, d.h. aus Sicht des Patienten von links nach rechts (bzw. umgekehrt) verläuft.

Erfindungsgemäß wird die gestellte Aufgabe dadurch gelöst, dass die maxillare und/oder mandibulare Miniplastschiene zumindest ein Montagemittel aufweist. Entgegen bekannten Ausführungsformen aus dem Stand der Technik wird nunmehr jedoch das Positionierungsmittel derart ausgeführt, dass es an dem an der Miniplastschiene befindlichen Montagemittel befestigt werden kann. Zur Ermöglichung verschiedener relativer Stellungen zwischen der mandibularen und der maxillaren Miniplastschiene ist weiterhin erfindungsgemäß vorgesehen, dass das am Montagemittel befestigte Positionierungsmittel ausgetauscht werden kann durch ein anderes in der Geometrie abweichendes Positionierungsmittel. Das heißt, dass jedes Positionierungsmittel, welches zur Befestigung am Montagemittel bestimmt ist, eine individuelle relative Stellung der Miniplastschienen zueinander bewirken kann.

Entsprechend der Möglichkeit, das am Montagemittel befestigte Positionierungsmittel austauschen zu können, folgt hieraus zwangsläufig, dass ebenso lediglich ein einzelnes Positionierungsmittel vorhanden sein kann, welches zwar prinzipiell austauschbar ist, hierzu jedoch kein zweites abweichendes Positionierungsmittel zum Austausch zur Verfügung steht.

Grundgedanke der vorliegenden Erfindung ist es, dass nicht mehr durch eine komplexe Einstellvorrichtung eine Anpassung an die relative Einstellung erfolgt, sondern vielmehr lediglich das Positionierungsmittel in einfacher Weise an einem Montagemittel befestigt wird und bei Bedarf kurzerhand ausgetauscht wird. Somit kann die Komplexität auf ein Minimum reduziert werden, wie auch eine besonders leichte Reinigung möglich ist. Insofern entfällt insbesondere die Notwendigkeit, die Miniplastschienen nach jedem Gebrauch chemisch zu reinigen. Vielmehr genügt es in vielen Fällen diese lediglich abzuspülen und nur gelegentlich der chemischen Reinigung zu unterziehen.

Weiterhin vorteilhaft ist bei dieser Ausführungsform, dass nach Ermittlung der erforderlichen relative. Stellung vom behandelnden Arzt bzw. Zahnarzt sodann die hierzu entsprechende Positionierungsmittel bereitgestellt werden können. Somit können im Fall der erforderlichen Änderung der relativen Stellung die hierzu notwendigen Positionierungsmittel in einfacher Weise auf dem Postweg vom Arzt zum Patienten gebracht werden. Eine fehlerhafte Einstellung kann hierbei nicht erfolgen und der Patient kann die erforderlichen Positionierungsmittel eigenständig am Montagemittel befestigen. Hingegen erfordert der Stand der Technik eine Einstellung durch den behandelnden Arzt bzw. Zahnarzt, so dass eine Veränderung der relativen Stellung durch den Patienten ohne Besuch des entsprechenden Arztes bzw. Zahnarztes praktisch ausgeschlossen ist. Sofern dies dennoch vom entsprechenden Patienten durchgeführt wird, ist dies mit erheblichem Risiko einer fehlerhaften Einstellung verbunden.

Erfindungsgemäß sind die Miniplastschienen mit einander gegenüberliegenden Anlageflächen ausgeführt. Dies führt beim Schließen des Gebisses zu einer entsprechenden Anlage der Miniplastschienen an den Anlageflächen aufeinander. Hierzu werden diese Anlageflächen vorzugsweise in der Okklusionsebene angeordnet und sind hier in vorteilhafterweise eben, d.h. planar, ausgeführt.

Zur Realisierung des Formschlusses zwischen dem maxillaren und mandibularen Positionierungsmittel zur Erzeugung einer definierten relativen Stellung der beiden Miniplastschienen zueinander in einer Querrichtung weist in besonders vorteilhafter Weise das maxillare Positionierungsmittel eine sich im Wesentlichen in Längsrichtung und näherungsweise in Hochrichtung erstreckende maxillare Führungsfläche auf. Analog weist hierzu das mandibulare Positionierungsmittel ebenso eine sich im Wesentlichen in Längsrichtung und näherungsweise in Hochrichtung erstreckende mandibulare Führungsfläche auf. Besonders vorteilhaft ist es hierbei, wenn die Führungsflächen zueinander komplementär ausgeführt sind. Somit kann beim Schließen des Gebisses der Formschluss zwischen den Führungsflächen und somit zwischen den Positionierungsmitteln realisiert werden. Durch die entsprechende Ausrichtung der Führungsflächen, welche quasi hochkant und näherungsweise parallel zu einer Mittelebene stehen, kann vorteilhaft die relative Stellung in Querrichtung definiert werden.

In weiterer besonders vorteilhafter Ausführungsform weist das maxillare Positionierungsmittel eine sich im Wesentlichen in Längsrichtung und vorwiegend in Hochrichtung erstreckende maxillare Positionierungsfläche auf. Hierzu analog weist das mandibulare Positionierungsmittel eine sich im Wesentlichen in Querrichtung und vorwiegend in Hochrichtung erstreckende mandibulare Positionierungsfläche auf. Gleichfalls ist es besonders vorteilhaft, die Positionierungsflächen zueinander komplementär auszuführen. Durch den Formschluss der Positionierungsflächen kann folglich die relative Stellung der Miniplastschienen zueinander in einer Längsrichtung definiert werden.

Besonders vorteilhaft ist es insofern, die Positionierungsmittel mit zugleich Führungsflächen und Positionierungsflächen auszuführen. Somit kann die relative Stellung der Miniplastschienen sowohl in Längsrichtung als auch in Querrichtung definiert werden.

Hierbei kann gleichfalls vorgesehen sein, dass vor einem Einsetzen der Miniplastschienen eine Anlage der Miniplastschienen unter Formschluss der Positionierungsmittel erfolgt, wobei in besonders vorteilhafter Weise vorgesehen ist, dass zunächst die Miniplastschienen jeweils auf den entsprechenden Zahnreihen aufgesetzt werden und der Formschluss sich erst durch eine Schließung des Gebisses ergibt. Hierbei ist es zunächst unerheblich, ob der Formschluss und somit die relative Stellung bereits bei anfänglichem Schließen des Gebisses erfolgt oder erst abschließend bei vollständiger Anlage der Miniplastschienen aneinander.

Hinsichtlich eines angenehmen Tragekomforts der Miniplastschienen und Gewährleistung der erforderlichen relativen Stellung ist es weiterhin besonders vorteilhaft, wenn die Führungsflächen abweichend von der Hochrichtung nach außen mit einem Winkel zwischen 1° und 10° geneigt sind. Diese Neigung der Führungsflächen stellt sicher, dass beim Schließen des Gebisses bei nicht exakt zentrischer Übereinstimmung der Miniplastschienen übereinander in der Querrichtung dennoch ein Schließen unvermindert erfolgen kann und die Führungsflächen aneinander gleiten können bis zum vollständigen Schließen des Gebisses mit entsprechender relativer Verschiebung der Miniplastschienen bis zur gewünschten Stellung in Querrichtung.

Gleichfalls ist es besonders vorteilhaft, wenn die Positionierungsflächen abweichend von der Hochrichtung nach vorne mit einem Winkel zwischen 10° und 40° geneigt sind. Entsprechend der potentiell größeren Abweichung der Miniplastschienen zueinander in der Längsrichtung bei geöffnetem Gebiss führt die entsprechende Neigung der Positionierungsflächen gleichfalls zu einer vorteilhaften Führung der Miniplastschienen zueinander beim Schließen des Gebisses. In welcher Richtung die Führungsfläche bzw. die Positionierungsfläche abweichend von der Hochrichtung zu neigen ist, ist für den Fachmann, bei Berücksichtigung der Schließmöglichkeit der Miniplastschienen ausgehend von einem geöffneten Gebiss, offensichtlich.

Erfindungsgemäß ist die Anordnung der Positionierungsmittel an den jeweiligen Miniplastschienen auf der linken und auf der rechten Seite im molaren Bereich an der Miniplastschiene vorgesehen. Das heißt, dass je Miniplastschiene zumindest zwei Positionierungsmittel vorhanden sein müssen. Dies ist dahingehend besonders vorteilhaft, dass der für die Positionierungsmittel erforderliche Bauraum bzw. der Platzbedarf im entsprechend molaren Bereich ohne besondere Beeinträchtigung des tragenden Patienten in Anspruch genommen werden kann. Hierbei ist es weiterhin besonders vorteilhaft, wenn die Positionierungsmittel sich auf der Außenseite der Zahnreihe befinden. Das heißt, dass sich die Positionierungsmittel im Backenbereich zwischen Zähnen und Wange befinden.

Hierbei ist es weiterhin besonders vorteilhaft zur Verwendung insbesondere bei der Schlafapnoe-Therapie, wenn das mandibulare Positionierungsmittel außerhalb und vor dem maxillaren Positionierungsmittel angeordnet ist. Im Fall der vorteilhaften Führungsflächen bedeutet dies, dass die maxillare Führungsfläche zwischen den Zahnreihen und dem mandibularen Positionierungsmittel angeordnet ist, welches folglich zwischen Wange und maxillarem Positionierungsmittel angeordnet ist.

Die Führung der mandibularen Miniplastschiene sowie der mandibularen Zahnreihe beim Schließen des Gebissen mit einer Bewegung in Bezug zur maxillaren Zahnreihe relativ nach vorne führt zu einer vorteilhaften Anordnung der mandibularen Positionsfläche vor der maxillaren Positionsfläche. Somit kann gleichfalls bei vorteilhaft geneigten Positionsflächen durch Schließen des Gebisses entlang der Positionierungsfläche ein Vorziehen der mandibularen Miniplastschiene erfolgen.

Eine besonders komfortable Trageweise als auch eine vorteilhafte Ausführungsform im Sinne einer besonders kostengünstigen Lösung als auch einer vorteilhaften Reinigung wird gewährleistet, wenn die Positionierungsmittel oberhalb der Okklusionsebene bzw. in Bezug auf die jeweilige Miniplastschiene oberhalb der Anlagefläche angeordnet sind. Das heißt, dass die maxillare Miniplastschiene mit dem maxillaren Positionierungsmittel vollständig oberhalb der Anlagefläche bzw. der Okklusionsebene angeordnet ist. Hingegen wird die mandibulare Miniplastschiene derart ausgeführt, dass sich deren mandibulares Positionierungsmittel oberhalb der Okklusionsebene bzw. oberhalb der Anlageflächen erstreckt und insofern auf Höhe der maxillaren Miniplastschiene angeordnet ist.

Eine besonders kostengünstige Ausführung sowie zugleich leichte Reinigung wird gewährleistet, wenn bei der Okklusionsschienenanordnung entweder die maxillare oder die mandibulare Miniplastschiene das Positionierungsmittel integral umfasst. Im Fall von zwei vorhandenen Positionierungsmitteln je Miniplastschiene bedeutet dies die Zuordnung der beiden zugehörigen Positionierungsmittel zur entsprechenden Miniplastschiene als ein integrales Bauteil. Hierdurch können sowohl die Herstellkosten gesenkt werden, als auch insbesondere durch die integrale Bauweise keine unnötigen Fugen oder Spalten entstehen und somit Schmutzablagerungen minimiert werden. Weiterhin ist es vorteilhaft, eine integrierte Bauweise zu wählen, als dass die Stabilität des Positionierungsmittels an der entsprechenden Miniplastschiene vorteilhaft beeinflusst werden kann.

Weiterhin ist es besonders vorteilhaft, wenn die Miniplastschiene entsprechend aus einem einzigen Material als ein einziges Bauteil hergestellt wird. Hiervon unbenommen sind jedoch Ausführungsformen, welche beispielsweise eingebettet in die Miniplastschiene Verstärkungselemente, wie beispielsweise Drahtbügel, aufweisen, oder bei denen die Zahnaufnahme zur Positionierung der Miniplastschiene auf der entsprechenden Zahnreihe von einem abweichenden Material als der eigentliche Träger der Miniplastschiene gebildet wird, welcher zumindest das Positionierungsmittel integral umfassen sollte.

Bezüglich der erfindungsgemäß vorhandenen auswechselbaren sich gegenseitig ersetzbaren Positionierungsmittel ist es besonders vorteilhaft, wenn diese relativ zum Montagemittel in Längsrichtung betrachtet verschieden zueinander angeordnete Positionierungsflächen aufweisen. Das heißt, dass die Variation der relativen Stellung der Miniplastschienen zueinander dadurch realisiert werden kann, dass die jeweiligen Positionierungsmittel entsprechende individuelle Abstände von der Positionierungsfläche zum Montagemittel aufweisen. Folglich wird durch einen Tausch der Positionierungsmittel eine Änderung der Positionierungsflächen bewirkt und somit eine Variation der relativen Stellung in Längsrichtung.

Zur Ausführung des Montagemittels ist es erfindungsgemäß vorgesehen, einen in der Miniplastschiene eingegossenen Zentrierstift zu verwenden. Somit kann der Zentrierstift zugleich eine Zentrierung des Positionierungsmittels bewirken als auch eine Befestigung für das Positionierungsmittel ermöglichen. Entsprechend weist das auswechselbare Positionierungsmittel eine zum Zentrierstift komplementäre Zentrieraufnahme zur Montage an der Miniplastschiene auf. Hierbei kann der Zentrierstift beispielsweise ein ausgestanztes Blechteil sein, welches in der Miniplastschiene durch beispielsweise Eingießen verankert sein kann.

Entsprechend der Ausführung des Positionierungsmittels mit einer Zentrieraufnahme werden die Positionierungsflächen zur Realisierung verschiedener relativer Stellungen der Miniplastschienen zueinander mit verschiedenen Abständen zur Zentrieraufnahme ausgeführt.

Unter Berücksichtung einer geneigten Positionierungsfläche und insbesondere bei Vorhandensein einer leicht geneigten Führungsfläche und dem zu berücksichtigenden Trageverhalten im Mund ist es erfindungsgemäß vorgesehen, dass das auswechselbare Positionierungsmittel in der Gestalt einer Finne ausgeführt ist. Hierbei kann diese mit einer Unterseite auf der Anlagefläche der zugehörigen Miniplastschiene zur Anlage kommen, wobei zumindest ein Abschnitt einer Seitenflanke die Führungsfläche bildet sowie zumindest ein Abschnitt einer Vorderkante die Positionierungsfläche erzeugt.

Unter Berücksichtigung der zu erwartenden Baugröße der Miniplastschienen und dem vorhandenen - ohne Komforteinschränkungennutzbaren Bauraum ist es besonders vorteilhaft, wenn das auswechselbare Positionierungsmittel eine Höhe über der Anlagefläche zwischen 5 mm und 30 mm, insbesondere zwischen 12 mm und 18 mm, aufweist. Hierbei kann das auswechselbare Positionierungsmittel die jeweils andere Miniplastschiene geringfügig überragen. Somit werden eine zuverlässige Führung und ein zuverlässiger Formschluss zwischen dem maxillaren und dem mandibularen Positionierungsmittel gewährleistet.

Die Formgebung der Führungsfläche ist zunächst unerheblich, sofern ein entsprechender Formschluss beim Schließen des Gebisses und Zuordnung der Miniplastschienen aufeinander ermöglicht wird. Insofern kann die Führungsfläche sowohl gewölbt oder eben sein. Unter Berücksichtigung der regulären Formgebung von Miniplastschienen und der Anordnung der Positionierungsmittel an den Miniplastschienen sowie unter Berücksichtigung einer geringfügigen Neigung abweichend von der Hochrichtung sind die Führungsflächen vorzugsweise weitgehend eben oder mit sehr geringer Wölbung auszuführen.

Die Gestaltung der Positionierungsflächen ist gleichfalls zunächst unerheblich, sofern beim Schließen des Gebisses ein entsprechender Formschluss zwischen den Positionierungsflächen ermöglicht wird. Diese können ebenso gewölbt sein, als auch eben ausgeführt werden. Bei einer gewölbten Ausführungsform der Positionierungsflächen führt in der Regel eine Schließbewegung des Gebisses bei nicht exakt übereinstimmender relativer Position der Miniplastschienen in Längsrichtung zu einer zunächst voreilenden und abschließend langsameren Vorwärtsbewegung des entsprechend vorne liegenden Positionierungsmittels samt zugehöriger Miniplastschiene. Nachteilig ist jedoch die komplexere Formgebung zur Realisierung komplementärer Formen zwischen den mandibularen und maxillaren Positionierungsflächen insbesondere unter Berücksichtigung der Auswechselbarkeit des Positionierungsmittels und folglich eignet sich hierbei besonders eine ebene Gestaltung.

In den nachfolgenden Figuren werden eine vorteilhafte Ausführungsform einer erfindungsgemäßen Okklusionsschienenanordnung sowie mögliche Variationen für das auswechselbare Positionierungsmittel skizziert.

Es zeigen:
- Fig. 1: ein exemplarisches Ausführungsbeispiel einer erfindungsgemäßen Okklusionsschienenanordnung in perspektivischer Ansicht;
- Fig. 2: die maxillare Miniplastschiene zur Ausführung aus Fig. 1;
- Fig. 3: die mandibulare Miniplastschiene zur Ausführung aus Fig. 1;
- Fig. 4: die Okklusionsschienenanordnung aus Fig. 1 in Seitenansicht;
- Fig. 5: die maxillare Miniplastschiene aus Fig. 4;
- Fig. 6: das auswechselbare Positionierungsmittel zu Fig. 4;
- Fig. 7: die mandibulare Miniplastschiene zu Fig. 4;
- Fig. 8: einen Schnitt durch die Okklusionsschienenanordnung;
- Fign. 9: verschiedene Positionierungsmittel für verschiedene relative Stellungen;
- Fign. 10: alternative Ausführungsformen der Positionierungsfläche.

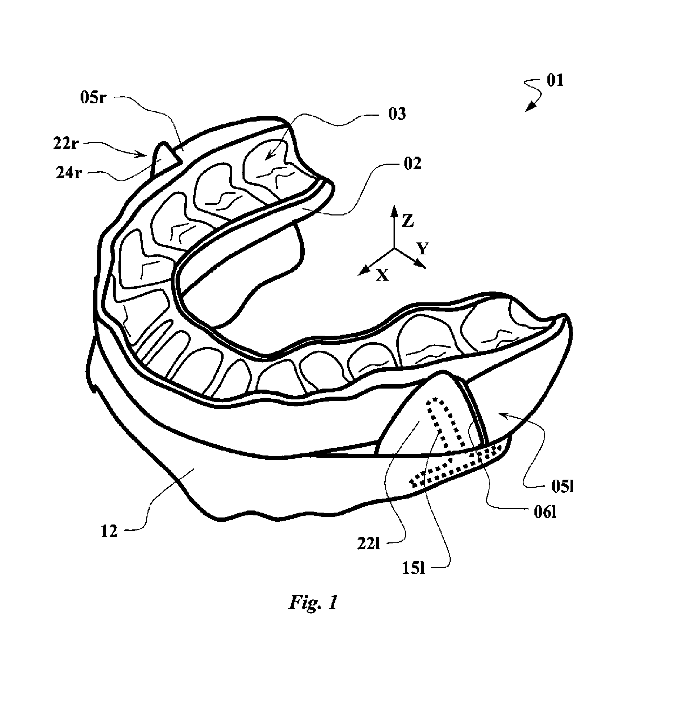

In der **Figur 1** wird eine beispielhafte Okklusionsschienenanordnung skizziert. Zu erkennen ist der Aufbau mit der maxillaren Miniplastschiene 02 sowie auf der unteren Seite mit der mandibularen Miniplastschiene 12. Hierbei weist die maxillare Miniplastschiene 02 eine entsprechende Zahnaufnahme 03 zur Anordnung auf der maxillaren Zahnreihe auf.

Analog hierzu weist entsprechend die mandibulare Miniplastschiene 12 eine Zahnaufnahme 13 (unterhalb verdeckt liegend) zur Anordnung der mandibularen Miniplastschiene 12 auf der mandibularen Zahnreihe auf.

Jede dieser Miniplastschienen 02, 12 weist hierbei beidseitig im molaren Bereich ein Positionierungsmittel 05 bzw. 22 auf. Zu erkennen ist bereits, dass das maxillare Positionierungsmittel 05r auf der rechten Seite sowie 051 auf der linken Seite einen integralen Bestandteil der maxillaren Miniplastschiene 02 bildet. Hingegen wird das mandibulare Positionierungsmittel 22 als auswechselbares Positionierungsmittel 22r für die rechte Seite sowie 221 für die linke Seite ausgeführt. Dieses ist jeweils auf einem Montagemittel in Form eines Zentrierstifts 15r bzw. 151 angebracht.

Jedes der Positionierungsmittel 05, 22 weist Führungsflächen 07, 24 auf. Diese dienen hierbei der Zentrierung und Führung der Miniplastschienen 02, 12 zueinander in der Querrichtung (Y). Die Positionierung in der Längsrichtung (X) wird mittels der Positionierungsflächen 06 sowie gegenüberliegend 23 realisiert.

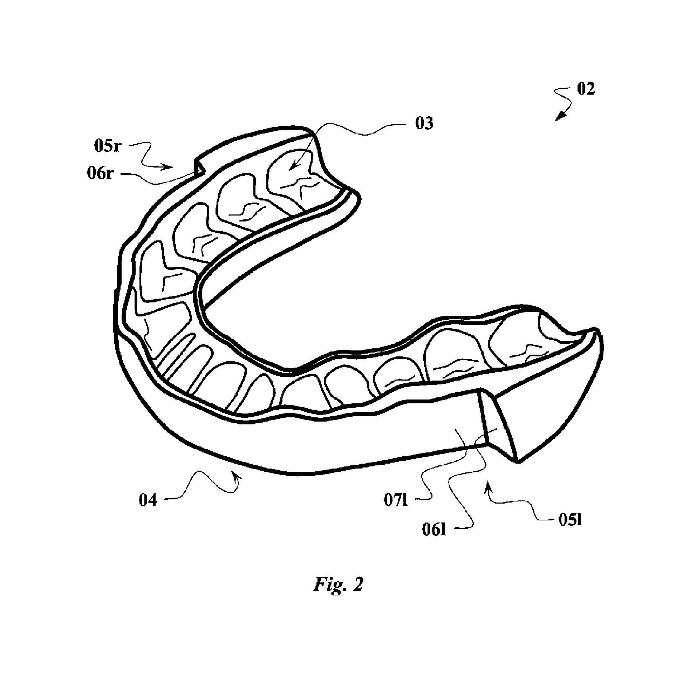

In der **Figur 2** wird hierzu nochmals die maxillare Miniplastschiene 02 skizziert. Zu erkennen ist wiederum die Anordnung der Zahnaufnahme 03 zur Anbringung der maxillaren Miniplastschiene 02 auf der maxillaren Zahnreihe des Patienten. Weiterhin sind die Positionierungsmittel 05r und 051 im mularen Bereich mit jeweils einer Führungsfläche 07 sowie einer Positionierungsfläche 06 zu erkennen. Unterseitig (verdeckt liegend) befindet sich die Anlagefläche 04, welche hier eine ebene Form aufweist und zur Anlage an der jeweils anderen Miniplastschiene 12 bestimmt ist.

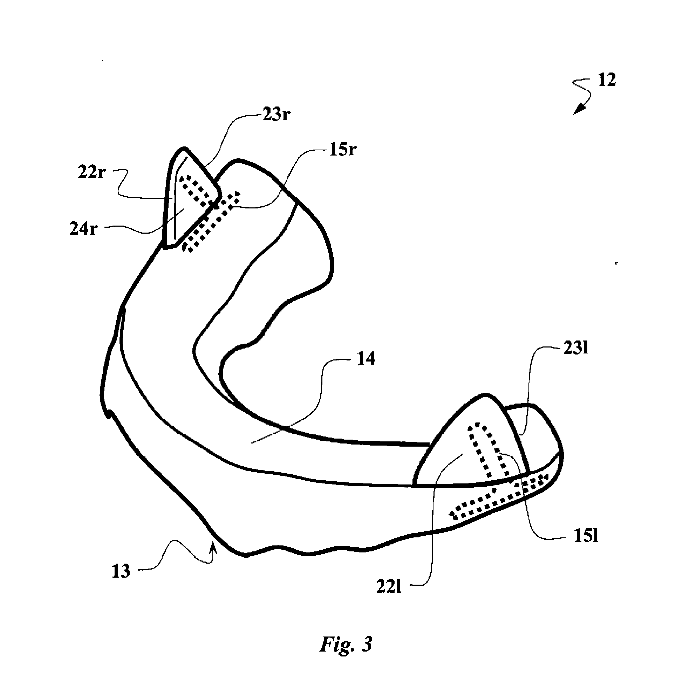

Die **Figur 3** skizziert die mandibulare Miniplastschiene 12 aus Fig. 1, bei welcher sich (wie bei Fig. 1) die Zahnaufnahme 13 untenliegend (verdeckt) befindet. Die dargestellte Oberseite bildet die Anlagefläche 14, welche gleichfalls eben ausgeführt ist und zur Anlage an der maxillaren Anlagefläche 04 der maxillaren Miniplastschiene 02 bestimmt ist.

Die relative Stellung zwischen den Miniplastschienen 02, 12 wird hier mittels auswechselbaren Positionierungsmitteln 22r, 221 realisiert, welche jeweils eine zum maximalen Positionierungsmittel 05 komplementäre Führungsfläche 24 sowie eine entsprechende komplementäre Positionierungsfläche 23 aufweisen. Zu erkennen ist weiterhin die skizzierte Befestigungsweise der Positionierungsmittel 22 auf der mandibularen Miniplastschiene 12, welche mittels im Grundkörper der Miniplastschiene verankerter Zentrierstifte 151, 15r erfolgt. Hierbei stellt sich das auswechselbare Positionierungsmittel 22 in Art einer Finne dar, bei der die zur Mitte hin weisende Fläche die Führungsfläche 24 und die nach hinten liegende Vorderkante der Finne die Positionierungsfläche 23 bildet.

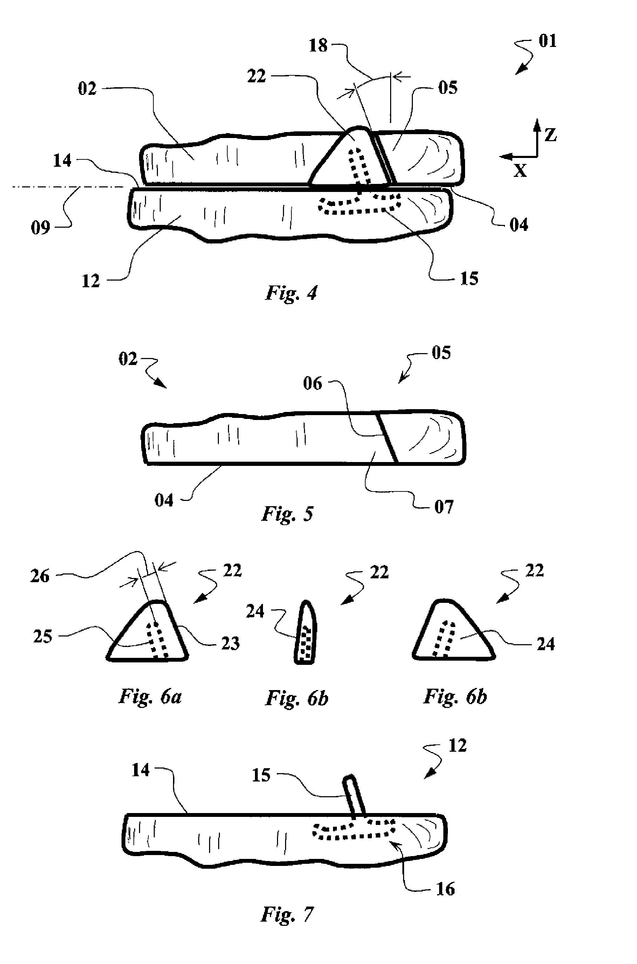

In der **Figur 4** wird nochmals die Okklusionsschienenanordnung 01 aus Fig. 1 in der Seitenansicht skizziert. Zu erkennen ist die Anordnung der Anlageflächen 04 der maxillaren Miniplastschiene 02 gegenüberliegend der Anlagefläche 14 der mandibularen Miniplastschiene 12, welche im Wesentlichen bei geschlossenem Gebiss in der Okklusionsebene 09 liegen. Hierbei ist des Weiteren zu erkennen, dass sich das maxillare Positionierungsmittel 05 der maxillaren Miniplastschiene 02 vollständig oberhalb der Anlagefläche 04 befindet, während hingegen das mandibulare Positionierungsmittel 22 oberhalb der entsprechenden Anlagefläche 14 auf der mandibularen Miniplastschiene 12 angeordnet ist. Weiterhin erkennbar ist die Gestaltung der Positionierungsflächen 06, 23, welche zunächst einmal eben gestaltet sind, jedoch gegenüber der Hochrichtung (Z) nach vorne geneigt ausgeführt sind. Hierbei ist des Weiteren (vgl. Fig. 1) zu berücksichtigen, dass das mandibulare Positionierungsmittel 22 sich außerhalb und vor dem maxillaren Positionierungsmittel 05 befindet. Somit wird sichergestellt, dass beim Schließen des Gebisses ein Vorziehen der mandibularen Miniplastschiene 12 und somit des Unterkiefers zur wirksamen Schlafapnoe-Therapie erfolgt.

In der **Figur 5** wird ergänzend zu Fig. 4 nochmals die maxillare Miniplastschiene 02 in der Seitenansicht skizziert, mit der in Querrichtung (Y) und Hochrichtung (Z) ausgerichteten Positionierungsfläche 06 sowie der in Längsrichtung (X) und Hochrichtung (Z) ausgerichteten Führungsfläche 07 des maxillaren Positionierungsmittels 05.

Die **Figur 6** skizziert in Ergänzung zu Fig. 4 das auswechselbare Positionierungsmittel 22 in Art einer Finne mit einer Zentrieraufnahme 25 zur Anbringung des Positionierungsmittels 22 auf entsprechend zugehörigem Zentrierstift 15. Hierbei bildet eine Seitenfläche der Finne als Positionierungsmittel 22 die Führungsfläche 24, wobei eine Vorderkante der Finne die Positionierungsfläche 23 des Positionierungsmittels 22 bildet. Zu erkennen ist ebenso der zur Festlegung der relativen Stellung der beiden Miniplastschienen 02, 12 zueinander maßgebliche Abstand 26 zwischen der Positionierungsfläche 23 und der Zentrieraufnahme 25.

Die **Figur 7** zeigt ergänzend zu Fig. 4 die mandibulare Miniplastschiene 12 jedoch ohne deren zugehörigem Positionierungsmittel 22. Zu erkennen ist das Montagemittel als Zentrierstift 15, welcher über der Anlagefläche 14 hervorsteht und hierbei mit einer Verankerung 16 in der Miniplastschiene 12 eingebettet ist.

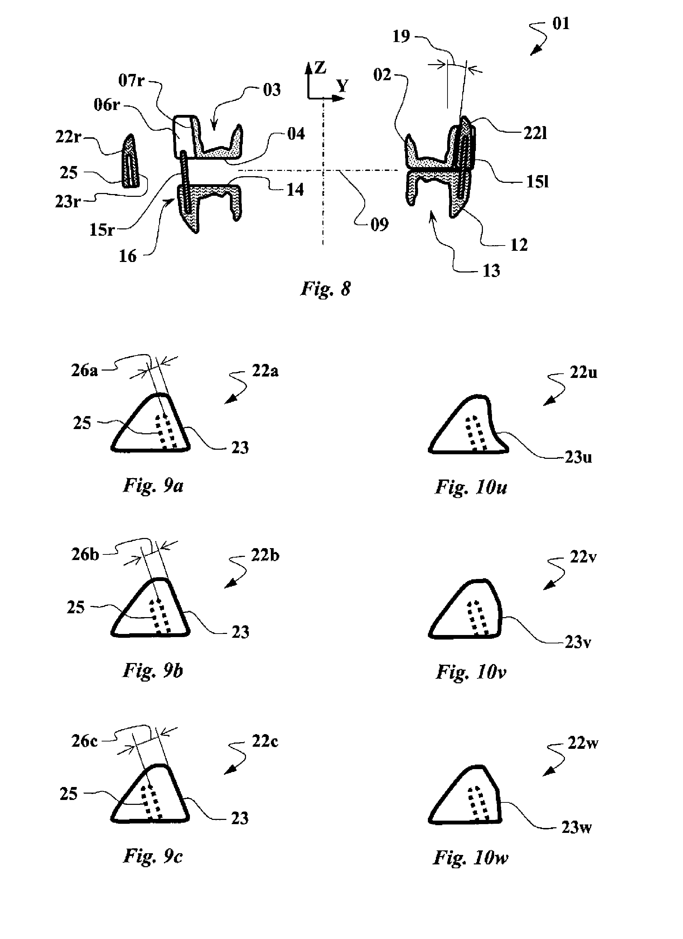

Die **Figur 8** skizziert ergänzend einen Schnitt durch die Okklusionsschienenanordnung 01, wobei diese auf der linken Seite als Explosionsdarstellung skizziert ist und rechter Hand in aufeinanderliegender Stellung. Zu erkennen ist wiederum die Anordnung der maxillaren Miniplastschiene 02 mit der maxillaren Zahnaufnahme 03 sowie gegenüberliegend der mandibularen Miniplastschiene 12 mit der entsprechenden Zahnaufnahme 13. Diese Miniplastschienen 02, 12 weisen jeweils entsprechende Anlageflächen 04, 14 auf, welche bei geschlossenem Biss in der Okklusionsebene 09 zur Anlage kommen. Die relative Führung in Querrichtung (Y) wird mittels der Führungsflächen 07 in Kontakt mit der Führungsflächen 24 sichergestellt. Diese Führungsflächen 07, 24 sind hierbei gegenüber der Hochrichtung (Z) nach außen um einen Neigungswinkel 19 geneigt. Somit wird sichergestellt, dass beim Schließen des Gebisses sich die Führungsflächen 07, 24 zueinander finden, ohne dass es zu einem Aufstoßen des mandibularen Positionierungsmittels 22 an der Anlagefläche 04 der maxillaren Miniplastschiene 02 kommt.

Weiterhin zu erkennen ist die Befestigungsweise des auswechselbaren Positionierungsmittels 22 an der mandibularen Miniplastschiene 12 durch Aufsetzen der Zentrieraufnahme 25 auf den Zentrierstift 15.

Die **Figurenfolge 9** skizziert nunmehr die erfindungswesentliche Möglichkeit der Relativanordnung zwischen der maxillaren Miniplastschiene 02 und der mandibularen Miniplastschiene 12. Hierbei wird jeweils der relevante Abstand 26 zwischen der Positionierungsfläche 23 und der Zentrieraufnahme 25 variiert mit einem geringen Abstand 26a in Fig. 9a, einem mittleren Abstand 26b in Fig. 9b und einem größeren Abstand 26c in Fig. 9c.

Abschließend skizzieren die **Fign. 10** alternative Ausführungsformen für die Positionierungsfläche 23 des Positionierungsmittels 22, wobei naheliegend ist, dass die komplementäre Positionierungsfläche 06 eine entsprechende Formgebung aufweisen sollte. Zu erkennen ist die konkave Formgebung der Positionierungsfläche 23u in Fig. 10u, der konvexen Positionierungsfläche 23v in Fig. 10v sowie einer abgewinkelten Positionierungsfläche 23w in Fig. 10w.

## Patentansprüche

1. Okklusionsschienenanordnung (01), insbesondere zur Schlafapnoe-Therapie, mit einer auf der maxillaren Zahnreihe anordnenbaren maxillaren Miniplastschiene (02) und einer auf der mandibularen Zahnreihe anordnenbaren mandibularen Miniplastschiene (12), wobei die maxillare Miniplastschiene (02) gegen die mandibulare Miniplastschiene (12) zur Anlage gebracht werden kann und wobei die Miniplastschienen (02, 12) mit ebenen, einander im Wesentlichen in der Okklusionsebene (09) gegenüber liegenden Anlageflächen (04, 14) ausgeführt sind, umfassend zumindest ein maxillares Positionierungsmittel (05) und zumindest ein mandibulares Positionierungsmittel (22), wobei durch Formschluss zwischen dem maxillaren und dem mandibularen Positionierungsmittel (05, 22) die relative Stellung der Miniplastschienen (02, 12) zueinander in Längsrichtung (X) und/oder in Querrichtung (Y) definierbar ist, und wobei auf der linken und auf der rechten Seite im molaren Bereich jeweils ein Positionierungsmittel (05r, 051, 22r, 221) je Miniplastschiene (02, 12) angeordnet ist,
und wobei die maxillare und/oder mandibulare Miniplastschiene (02, 12) zumindest ein Montagemittel (15) aufweist, wobei zumindest zwei verschiedene Positionierungsmittel (22a, 22b, 22c) sich gegenseitig ersetzend am gleichen Montagemittel (15) befestigt werden können, wodurch zumindest zwei verschiedene relative Stellungen zwischen den Miniplastschienen (02, 12) definierbar sind,
und wobei das Montagemittel ein Zentrierstift (15) ist, wobei das auswechselbare Positionierungsmittel (22) eine zum Zentrierstift (15) komplementäre Zentrieraufnahme (25) zur Montage an der Miniplastschiene (12) aufweist, und wobei das auswechselbare Positionierungsmittel (22) durch Aufsetzen der Zentrieraufnahme (25) auf den Zentrierstift (15) befestigbar ist, und wobei der Zentrierstift (15) zugleich eine Zentrierung des Positionierungsmittels (22) und eine Befestigung des Positionierungsmittels (22) ermöglicht, und wobei das auswechselbare Positionierungsmittel (22) in der Gestalt einer Finne ausgeführt ist, wobei die Unterseite auf der Anlagefläche (14) der zugehörigen Miniplastschiene (12) zur Anlage kommt und ein Abschnitt einer Seitenflanke die Führungsfläche (24) und ein Abschnitt einer Vorderkante die Positionierungsfläche (23) bildet,
**dadurch gekennzeichnet, dass** der Zentrierstift ein in der Miniplastschiene eingegossen verankerter Zentrierstift ist, der über die Anlagefläche hervorsteht.

2. Okklusionsschienenanordnung (01) nach Anspruch 1,
**dadurch gekennzeichnet,**
**dass** das maxillare Positionierungsmittel (05) eine sich im Wesentlichen in Längsrichtung (X) und näherungsweise in Hochrichtung (Z) erstreckende maxillare Führungsfläche (07) und das mandibulare Positionierungsmittel (22) eine sich im Wesentlichen in Längsrichtung (X) und näherungsweise in Hochrichtung (Z) erstreckende mandibulare, insbesondere komplementäre, Führungsfläche (24) aufweist, wobei durch Formschluss der Führungsflächen (07, 24) die relative Stellung der Miniplastschienen (02, 12) zueinander in Querrichtung (Y) definierbar ist.

3. Okklusionsschienenanordnung (01) nach Anspruch 1 oder 2,
**dadurch gekennzeichnet,**
**dass** das maxillare Positionierungsmittel (05) eine sich im Wesentlichen in Querrichtung (Y) und vorwiegend in Hochrichtung (Z) erstreckende maxillare Positionierungsfläche (06) und das mandibulare Positionierungsmittel (22) eine sich im Wesentlichen in Querrichtung (Y) und vorwiegend in Hochrichtung (Z) erstreckende mandibulare, insbesondere komplementäre, Positionierungsfläche (23) aufweist, wobei durch Formschluss der Positionierungsflächen (06, 23) die relative Stellung der Miniplastschienen (02, 12) zueinander in Längsrichtung (X) definierbar ist.

4. Okklusionsschienenanordnung (01) nach Anspruch 2 oder 3,
**dadurch gekennzeichnet,**
**dass** die Führungsfläche (07, 24) abweichend zur Hochrichtung (Z) nach außen, insbesondere mit einem Winkel (19) zwischen 1° und 10°, und/oder die Positionierungsfläche (06, 23) abweichend zur Hochrichtung (Z) nach vorne, insbesondere mit einem Winkel (18) zwischen 10° und 40°, geneigt ist.

5. Okklusionsschienenanordnung (01) nach einem der Ansprüche 1 bis 4,
**dadurch gekennzeichnet,**
**dass** das mandibulare Positionierungsmittel (22) außerhalb und vor dem maxillaren Positionierungsmittel (05) angeordnet ist.

6. Okklusionsschienenanordnung (01) nach einem der Ansprüche 1 bis 5,
**dadurch gekennzeichnet,**
**dass** das maxillare und das mandibulare Positionierungsmittel (05, 22) im Wesentlichen oberhalb der jeweiligen Anlagefläche (04, 14) der maxillaren bzw. mandibularen Miniplastschiene (02, 12) angeordnet ist.

7. Okklusionsschienenanordnung (01) nach einem der Ansprüche 1 bis 6,
**dadurch gekennzeichnet,**
**dass** die maxillare oder die mandibulare Miniplastschiene (02, 12) das Positionierungsmittel (05) integral umfasst.

8. Okklusionsschienenanordnung (01) nach einem der Ansprüche 1 bis 7,
**dadurch gekennzeichnet,**
**dass** die auswechselbaren, sich gegenseitig ersetzenden Positionierungsmittel (22) relativ zum Montagemittel (15) in Längsrichtung verschieden zueinander angeordnete Positionierungsflächen (23) aufweisen.

9. Okklusionsschienenanordnung (01) nach Anspruch 8,
**dadurch gekennzeichnet,**
**dass** die auswechselbaren, sich gegenseitig ersetzenden Positionierungsmittel (22a, 22b, 22c) verschiedene Abstände (26) der Positionierungsfläche (23) zur Zentrieraufnahme (25) aufweisen.

10. Okklusionsschienenanordnug (01) nach einem der Ansprüche 1 bis 9,
**dadurch gekennzeichnet,**
**dass** das auswechselbare Positionierungsmittel (22) eine Höhe über der Anlagefläche (14) zwischen 5 mm und 30 mm, insbesondere zwischen 12 mm und 18 mm, aufweist.

## Claims

1. An occlusal splint arrangement (01), in particular for sleep apnea therapy, comprising a maxillary miniplast splint (02) that can be arranged on the maxillary row of teeth and a mandibular miniplast splint (12) that can be arranged on the mandibular row of teeth, wherein the maxillary miniplast splint (02) can be brought to bear against the mandibular miniplast splint (12), and wherein the miniplast splints (02, 12) are realized with even contact surfaces (04, 14) that substantially oppose each other in the occlusal plane (09) comprising at least one maxillary positioning means (05) and at least one mandibular positioning means (22), wherein the relative position of the miniplast splints (02, 12) toward each other can be defined in the longitudinal direction (X) and/or in the transverse direction (Y) by a form fit between the maxillary and the mandibular positioning means (05, 22), and wherein one positioning means (05r, 051, 22r, 221) per miniplast splint (02, 12) is arranged on the left and on the right side in the molar area, respectively,
and wherein the maxillary and/or mandibular miniplast splint (02, 12) comprises at least one mounting means (15), wherein at least two different positioning means (22a, 22b, 22c) can be secured as mutual replacements to the same mounting means (15), whereby at least two different relative positions can be defined between the miniplast splints (02, 12),
and wherein the mounting means is a centering pin (15), the exchangeable positioning means (22) having a centering recess (25) that is complementary to the centering pin (15) for mounting to the miniplast splint (12), and the exchangeable positioning means (22) being attachable by placing the centering recess (25) on the centering pin (15), and the centering pin (15) simultaneously allowing a centering of the positioning means (22) and an attachment of the positioning means (22), and the exchangeable positioning means (22) being formed in the shape of a fin, the bottom side coming to bear on the contact surface (14) of the associated miniplast splint (12) and a portion of a lateral flank forming the guiding surface (24) and a portion of a front edge forming the positioning surface (23),
**characterized in that**
the centering pin is anchored by being cast into the miniplast splint and protrudes beyond the contact surface.

2. The occlusal splint arrangement (01) according to claim 1,
**characterized in that**
the maxillary positioning means (05) has a maxillary guiding surface (07) that extends substantially in the longitudinal direction (X) and approximately in the vertical direction (Z), and the mandibular positioning means (22) has a mandibular, in particular complementary, guiding surface (24) that extends substantially in the longitudinal direction (X) and approximately in the vertical direction (Z), the relative position of the miniplast splints (02, 12) toward each other being definable in the transverse direction (Y) by a form fit of the guiding surfaces (07, 24).

3. The occlusal splint arrangement (01) according to claim 1 or 2,
**characterized in that**
the maxillary positioning means (05) has a maxillary positioning surface (06) that extends substantially in the transverse direction (Y) and mainly in the vertical direction (Z), and the mandibular positioning means (22) has a mandibular, in particular complementary, positioning surface (23) that extends substantially in the transverse direction (Y) and mainly in the vertical direction (Z), the relative position of the miniplast splints (02, 12) toward each other being definable in the longitudinal direction (X) by a form fit of the positioning surfaces (06, 23).

4. The occlusal splint arrangement (01) according to claim 2 or 3,
**characterized in that**
the guiding surface (07, 24) is inclined to the outside deviating from the vertical direction (Z), in particular at an angle (19) of between 1° and 10°, and/or the positioning surface (06, 23) is inclined forward deviating from the vertical direction (Z), in particular at an angle (18) of between 10° and 40°.

5. The occlusal splint arrangement (01) according to any of the claims 1 to 4,
**characterized in that**
the mandibular positioning means (22) is arranged outside and in front of the maxillary positioning means (05).

6. The occlusal splint arrangement (01) according to any of the claims 1 to 5,
**characterized in that**
the maxillary and the mandibular positioning means (05, 22) are arranged substantially above the respective contact surface (04, 14) of the maxillary and mandibular miniplast splint (02, 12), respectively.

7. The occlusal splint arrangement (01) according to any of the claims 1 to 6,
**characterized in that**
the maxillary or the mandibular miniplast splint (02, 12) integrally comprises the positioning means (05).

8. The occlusal splint arrangement (01) according to any of the claims 1 to 7,
**characterized in that**
the exchangeable positioning means (22) that replace one another have positioning surfaces (23) that are arranged differently to one another in the longitudinal direction relative to the mounting means (15).

9. The occlusal splint arrangement (01) according to claim 8,
**characterized in that**
the exchangeable positioning means (22a, 22b, 22c) that replace one another have different distances (26) of the positioning surface (23) to the centering recess (25).

10. The occlusal splint arrangement (01) according to any of the claims 1 to 9,
**characterized in that**
the exchangeable positioning means (22) has a height above the contact surface (14) of between 5 mm and 30 mm, in particular between 12 mm and 18 mm.

## Revendications

1. Système de gouttière occlusale (01), en particulier pour le traitement de l'apnée de sommeil, comprenant une gouttière miniplast maxillaire (02) étant disposable sur la rangée de dents maxillaire et une gouttière miniplast mandibulaire (12) étant disposable sur la rangée de dents mandibulaire, la gouttière miniplast maxillaire (02) pouvant être mise en appui contre la gouttière miniplast mandibulaire (12) et les gouttières miniplast (02, 12) étant réalisées avec des surfaces d'appui (04, 14) planes qui sont substantiellement opposées l'une à l'autre dans le plan d'occlusion (09), comprenant au moins un moyen de positionnement maxillaire (05) et au moins un moyen de positionnement mandibulaire (22), la position relative des gouttières miniplast (02, 12) l'une par rapport à l'autre pouvant être définie dans le sens longitudinal (X) et/ou dans le sens transversal (Y) par une complémentarité de forme entre le moyen de positionnement maxillaire (05) et le moyen de positionnement mandibulaire (22), et un moyen de positionnement (05r, 051, 22r, 221) par gouttière miniplast (02, 12) étant disposé sur le côté gauche et sur le côté droit dans la région molaire,
et dans lequel la gouttière miniplast maxillaire (02) et/ou la gouttière miniplast mandibulaire (12) comporte au moins un moyen de montage (15), au moins deux moyens de positionnement (22a, 22b, 22c) différents pouvant être fixés au même moyen de montage (15) de manière à se remplacer mutuellement, ce qui permet de définir au moins deux positions relatives différentes entre les gouttières miniplast (02, 12),
et dans lequel le moyen de montage est une broche de centrage (15), le moyen de positionnement (22) échangeable ayant un évidement de centrage (25) complémentaire à la broche de centrage (15) pour le montage sur la gouttière miniplast (12) et le moyen de positionnement (22) échangeable pouvant être fixé en plaçant l'évidement de centrage (25) sur la broche de centrage (15), et la broche de centrage (15) permettant en même temps un centrage du moyen de positionnement (22) et une fixation du moyen de positionnement (22), et le moyen de positionnement (22) échangeable se présentant sous la forme d'un aileron, la face inférieure venant en appui contre la surface d'appui (14) de la gouttière miniplast (12) associée et une partie d'un bord latéral formant la surface de guidage (24) et une partie d'un bord avant formant la surface de positionnement (23),
**caractérisé en ce que**
la broche de centrage est une broche de centrage ancrée par coulée dans la gouttière miniplast, ladite broche de centrage faisant saillie sur la surface d'appui.

2. Système de gouttière occlusale (01) selon la revendication 1,
**caractérisé en ce que**
le moyen de positionnement maxillaire (05) comporte une surface de guidage maxillaire (07) qui s'étend substantiellement dans le sens longitudinal (X) et approximativement dans le sens de la hauteur (Z) et le moyen de positionnement mandibulaire (22) comporte une surface de guidage mandibulaire (24), en particulier complémentaire, qui s'étend substantiellement dans le sens longitudinal (X) et approximativement dans le sens de la hauteur (Z), la position relative des gouttières miniplast (02, 12) l'une par rapport à l'autre pouvant être définie dans le sens transversal (Y) par une complémentarité de forme des surfaces de guidage (07, 24).

3. Système de gouttière occlusale (01) selon la revendication 1 ou 2,
**caractérisé en ce que**
le moyen de positionnement maxillaire (05) comporte une surface de positionnement maxillaire (06) qui s'étend substantiellement dans le sens transversal (Y) et principalement dans le sens de la hauteur (Z) et le moyen de positionnement mandibulaire (22) comporte une surface de positionnement mandibulaire (23), en particulier complémentaire, qui s'étend substantiellement dans le sens transversal (Y) et principalement dans le sens de la hauteur (Z), la position relative des gouttières miniplast (02, 12) l'une par rapport à l'autre pouvant être définie dans le sens longitudinal (X) par une complémentarité de forme des surfaces de positionnement (06, 23).

4. Système de gouttière occlusale (01) selon la revendication 2 ou 3,
**caractérisé en ce que**
la surface de guidage (07, 24) est inclinée vers l'extérieur de manière à dévier par rapport au sens de la hauteur (Z), en particulier sous un angle (19) de 1° à 10°, et/ou la surface de positionnement (06, 23) est inclinée vers l'avant de manière à dévier par rapport au sens de la hauteur (Z), en particulier sous un angle (18) de 10° à 40°.

5. Système de gouttière occlusale (01) selon l'une quelconque des revendications 1 à 4,
**caractérisé en ce que**
le moyen de positionnement mandibulaire (22) est disposé en dehors et devant le moyen de positionnement maxillaire (05).

6. Système de gouttière occlusale (01) selon l'une quelconque des revendications 1 à 5,
**caractérisé en ce que**
le moyen de positionnement maxillaire (05) et le moyen de positionnement mandibulaire (22) sont disposés substantiellement au-dessus de la surface d'appui (04, 14) respective de la gouttière miniplast maxillaire (02) et de la gouttière miniplast mandibulaire (12).

7. Système de gouttière occlusale (01) selon l'une quelconque des revendications 1 à 6,
**caractérisé en ce que**
la gouttière miniplast maxillaire (02) ou la gouttière mandibulaire (12) comprend le moyen de positionnement (05) de manière intégrée.

8. Système de gouttière occlusale (01) selon l'une quelconque des revendications 1 à 7,
**caractérisé en ce que**
les moyens de positionnement échangeables (22), qui se remplacent mutuellement, comportent des surfaces de positionnement (23) qui sont disposées de manière différente l'une par rapport à l'autre dans le sens longitudinal relatif au moyen de montage (15).

9. Système de gouttière occlusale (01) selon la revendication 8,
**caractérisé en ce que**
les moyens de positionnement échangeables (22a, 22b, 22c), qui se remplacent mutuellement, présentent des distances (26) différentes entre la surface de positionnement (23) et l'évidement de centrage (25).

10. Système de gouttière occlusale (01) selon l'une quelconque des revendications 1 à 9,
**caractérisé en ce que**
le moyen de positionnement échangeable (22) présente une hauteur de 5 mm à 30 mm, en particulier de 12 mm à 18 mm, au-dessus de la surface d'appui (14).
